# EUROPÄISCHE PATENTSCHRIFT

(11) **EP 0 800 860 B1**
(45) Veröffentlichungstag und Bekanntmachung des Hinweises auf die Patenterteilung: **14.11.2001**
(21) Anmeldenummer: 97105180.0
(22) Anmeldetag: 27.03.1997
(51) Int. Cl.: B01J 2/16, A61K 9/16

(54) **Verfahren und Vorrichtung zur Agglomeration von hydrolyseempfindlichen Stoffen mittels Wasserdampf**
Process and device for the agglomeration of products sensitive to hydrolysis using steam
Procédé et installation pour l'agglomération des products sensibles à l'hydrolyse ou moyen de vapeur d'eau

(30) Priorität: 09.04.1996 DE 19614063; 12.06.1996 DE 19623410
(43) Veröffentlichungstag der Anmeldung: 15.10.1997
(73) Patentinhaber: BAYER AG, 51368 Leverkusen (DE)
(72) Erfinder: Seyffert, Ina, 51061 Köln (DE); Uhlemann, Hans, Dr., 42657 Solingen (DE); Walter, Reinhardt, Dr., 51375 Leverkusen (DE); Maasz, Joachim Martin, Dr., Station, New Jersey 07960 (US)

(56) Entgegenhaltungen:
- EP-A- 0 163 836
- EP-A- 0 202 076
- DE-A- 1 940 915
- US-A- 3 143 428

## Beschreibung

Die Erfindung betrifft ein spezielles Verfahren zur Agglomeration von schwer löslichen und hydrolyseempfindlichen Stoffen, insbesondere pharmazeutischen Wirkstoffen wie z.B. Acetylsalicylsäure (ASS), durch Verwendung von Wasserdampf. Ferner betrifft die Erfindung eine Vorrichtung zur Durchführung des Verfahrens.

Viele Stoffe lassen sich in Pulverform schlecht in Flüssigkeiten dispergieren und daher ist ihr Einsatz für Dispersionen oder Lösungen oftmals schwierig. Häufig wird außerdem ein gutes Fließverhalten und ein möglichst geringer Staubanteil gewünscht. Dies gilt auch für schwerlösliche Pulver, die sowohl hydrophile als auch hydrophobe Oberflächeneigenschaften aufweisen können. Werden Pulver für galenische Zubereitungen verwendet, ist es darüber hinaus häufig wünschenswert oder notwendig, Geschmacksverbesserungen oder -maskierungen zu erreichen oder den Kontakt mit anderen, unverträglichen Mischungskomponenten zu vermeiden bzw. zu minimieren.

Die Verbesserung der Benetzung, der Fließfähigkeit und die Verringerung des Staubanteils beim Einsatz von Pulvern werden üblicherweise durch die Granulation der betreffenden Pulver durch ein Verfahren der Aufbauagglomeration erreicht. Aufbauagglomerationsverfahren sind durch den Einsatz von Granulierflüssigkeit, üblicherweise Wasser oder wäßrige Lösungen gekennzeichnet. Aus EP 163 836 A1 ist beispielsweise ein Verfahren zur kontinuirlichen Herstellung von Granulaten bekannt, wobei das zu granulierende Produkt in flüssiger Form in ein Wirbelbett eingesprüht wird. Diese Verfahren sind also für die Verarbeitung hydrolyseempfindlicher Stoffe nicht anwendbar, da hierbei in der Regel eine Zersetzung des (Wirk)stoffes unter Bildung von (pharmazeutisch) unerwünschten Abbauprodukten auftritt. Werden deshalb nicht wäßrige organische Lösemittel verwendet, führt dies zu Lösemittelrestgehalten im Granulat, die ebenfalls nicht erwünscht oder unzulässig sind, Ebenfalls entsteht durch die Arbeit mit Lösemittel ein erhöhter Aufwand zur Verarbeitung.

US 3,143,428 offenbart ein Verfahren zur Agglomeration von pulverförmigen Materialien, die einen größeren Anteil an Zucker enthalten. Dieses Verfahren enthält einen ersten Schritt, bei dem die pulverförmigen Materialien befeuchtet werden und einen zweiten Schritt, bei dem die Partikel durch das Besprühen mit trockenem Dampf oder mit Luft in turbulente Bewegungen versetzt werden, um sie zu agglomerieren.

Es hat sich gezeigt, daß leicht dispergierbare Granulate erhalten werden, wenn durch das Agglomerationsverfahren zwischen den Primärpartikeln sehr kleine Feststoffbrücken hergestellt werden, die beim Redispergieren in eine Flüssigkeit in dieser leicht aufgelöst werden können und bevorzugt aus einem Material bestehen, das in der Flüssigkeit gut löslich ist. Dabei ist es für die gute Dispergierbarkeit der Granulate weiterhin vorteilhaft, wenn während der Agglomeration, d.h. im noch feuchten Zustand des Granulates, kompaktierende Kräfte vermieden werden, da sonst die Konstaktstellen zwischen den befeuchteten Partikeln vergrößert werden und größere, stabilere Feststoffbrücken entstehen.

Gegenstand der Erfindung ist ein Verfahren zur Agglomeration von hydrolyseempfindlichen Stoffen, insbesondere von ASS, dadurch gekennzeichnet, daß man das schwer lösliche ASS-Pulver zusammen mit mindestens einem leicht wasserlöslichen pulvrigen Bindemittel im freien Fall durch eine Wasserdampfatmosphäre bei Temperaturen zwischen 85°C und 105°C, vorzugsweise bei ca. 100°C, im wesentlichen ohne Einsatz kompaktierender Kräfte führt mit einer Verweildauer in der Dampfzone von ca. 0,5 bis 10 Sekunden, vorzugsweise 1 bis 3 Sekunden, und anschließend im weiteren freien Fall antrocknet, so daß sich an den Kontaktstellen zwischen den Partikeln des wasserunlöslichen Wirkstoffes und des wasserlöslichen Bindemittels aus den aufgrund der Kondensation entstandenen Flüssigkeitsbrücken, in denen Bindemittel gelöst ist, kleine Feststoffbrücken entstehen und in einem nachfolgenden Trocknungsvorgang in einem integrierten Wirbelbett bis zu einem Wassergehalt von weniger als 5 Gew.-%, vorzugsweise weniger als 1 Gew.-%, trocknet.

Unter 'kleinen Feststoffbrücken' wird dabei verstanden, daß die Feststoffbrücken eine mittlere Querabmessung (Durchmesser bzw. Dicke) von 1 µm bis 30 µm, vorzugsweise 5 bis 15 µm, haben sollen.

Dadurch, daß die Kontaktzeit des Wassers mit dem hydrolyseempfindlichen Pulver sehr kurz ist, und auch die Wärmebelastung der festen Teilchen mit maximal 100°C, vorzugsweise maximal 86°C, gering ist und nur sehr kurz erfolgt, treten während des erfindungsgemäßen Verfahrens keine wesentlichen Abbaureaktionen auf.

Das Anlösen der Partikeloberfläche des Bindemittels durch den kondensierten Wasserdampf erfolgt sehr schnell, die dabei entstehenden Lösungen bilden an den Kontaktstellen zwischen den Partikeln Flüssigkeitsbrücken bzw. ziehen auf die schwer löslichen, hydrolyseempfindlichen Partikel auf. Die nach der Agglomeration stattfindende sofortige Verdampfung des Wassers unter Bildung von Feststoffbrücken bzw. (teilweise) Umhüllungen der unlöslichen Wirkstoffteilchen mit den wasserlöslichen Hilfsstoffen gewährleistet die Stabilität auch bei hydrolyseempfindlichen Stoffen, wie ASS.

Mit der beschriebenen Methode ist bei Wahl geeigneter Verhältnisse von Wirkstoff und Hilfsmittel und günstiger Verfahrensbedingungen auch eine Art Mikroverkapselung bzw. ein Coating der wasserunlöslichen Stoffe möglich.

Für die Agglomeration bzw. (teilweise) Mikroverkapselung der schwer- bzw. unlöslichen Stoffe werden gut wasserlösliche, bindende Hilfsstoffe wie Polyvinylpyrrolidon (PVP), PVP-Derivate, Stärke, Stärke- und Zellulosederivate, Zucker, Zuckeralkohole wie Sorbitol, Xylitol, Zuckerderivate wie Maltodextrine, Isomaltose, Fruchtsäuren und deren wasserlösliche Salze wie Citrate oder Tartrate, Ascorbinsäure, Aminosäuren oder auch anorganische Salze wie Natriumsulfat verwendet.

Das Gewichtsverhältnis der schwer löslichen Wirkstoffe zu den wasserlöslichen bindenden Hilfsstoffen beträgt 1 bis 10 zu 10 bis 1, vorzugsweise 3 bis 8 zu 7 bis 2. Das zu agglomerierende Gemisch aus hydrolyseempfindlichem, unlöslichem Wirkstoff und wasserlöslichem Hilfsstoff wird vorteilhaft von oben gemeinsam mit dem Wasserdampf räumlich nahe in den Apparat eingebracht. Der Dampf kondensiert auf den kälteren Pulverteilchen, der Kondensatfilm löst das Bindemittel an, und die an den Kontaktstellen zwischen den angefeuchteten Teilchen gebildeten Flüssigkeitsbrücken werden zu Feststoffbrücken getrocknet. Im Falle eines großen Überschusses von wasserlöslichen Hilfsstoffen kann eine vollständige Abschirmung des hydrolyseempfindlichen Wirkstoffs durch das wasserlösliche Hilfsmittel erreicht werden. Das Verfahren kann auch so angewendet werden, daß ein gut wasserlöslicher Wirkstoff als Bindemittel wirkt.

Ein wesentliches Element des erfindungsgemäßen Verfahrens ist die Verwendung einer nahezu reinen Dampfatmosphäre zur direkten Befeuchtung der Partikeloberfläche. Bei der gleichzeitigen Anwesenheit von Luft müßten sonst vom Dampf zur Partikeloberfläche hin entsprechende Luftpolster durch Diffusion durchquert werden. Dadurch kann weniger Dampf auf dem Feststoff kondensieren, es kommt somit zu einem geringeren Grad der Agglomeration bzw. der Abschirmung des Wirkstoffes. So kann das Ziel der Behandlung des hydrolyseempfindlichen Pulvers nicht oder nur in unzureichendem Maße erfüllt werden.

Zur Durchführung des erfindungsgemäßen Verfahrens hat sich eine Vorrichtung bewährt, die aus einem Agglomerator mit einem geschlossenen Gehäuse besteht, an dessen oberen Ende eine Dosiervorrichtung für ein pulverförmiges Gut in Verbindung mit einem Aufgabetrichter angebracht ist, durch den ein im Agglomerator frei fallender Produktschleier von pulverförmigem Gut erzeugt wird. Außerdem ist die Vorrichtung im oberen Teil des Agglomerators mit Düsen zur Erzeugung von Dampfstrahlen ausgestattet, die den frei fallenden Produktschleier innerhalb einer Dampfzone zumindest teilweise einhüllen. Das erfindungsgemäße Kennzeichen dieser Apparatur besteht darin, daß sich an den unteren Teil des Agglomerators ein Wirbelbetttrockner derart anschließt, daß die agglomerierten Teilchen direkt in das Wirbelbett fallen. Der Wirbelbetttrockner ist also direkt in die Dampfstrahl-Agglomerationsapparatur integriert.

Vorteilhaft bestehen die Dampfstrahldüsen aus Rohren oder Bohrungen, die mit einem sich in Längsrichtung des Agglomerators erstreckenden Verteilerrohr verbunden sind, wobei zu beiden Seiten des Produktschleiers je ein Verteilerrohr angeordnet ist.

Ein wesentliches Element der erfindungsgemäßen Vorrichtung ist auch die Trennung der Dampfzone (Befeuchtungszone) von der Trocknungszone. Diese Trennung erfolgt gemäß einer Weiterentwicklung der Erfindung dadurch, daß im oberen Teil des Agglomerators ein die Dampfstrahldüsen einschließendes, beheiztes, doppelwandiges Schutzrohr angeordnet wird, durch dessen Mantel Abluft abgezogen wird. Auf diese Weise können am unteren Abschluß dieses Schutzrohrs alle durch den Wirbelbetttrockner in den Apparat eingebrachten Gasströme abgezogen werden.

Alternativ kann die Trennung der Dampfzone von der Trocknungszone auch dadurch erfolgen, daß in einem Abstand von 50 mm bis 300 mm vom unteren Ende der Dampfstrahldüsen-Verteilerrohre am Agglomeratorgehäuse ein Ringspalt mit einem Sammelkanal zur Entnahme der Abluft vorgesehen ist. Auf diese Weise kann ebenfalls die gesamte Abluft im Bereich zwischen Bedampfungs- und Trocknungsteil über den gesamten Apparateumfang gleichmäßig abgezogen werden.

Eine bevorzugte Weiterentwicklung der erfindungsgemäßen Vorrichtung besteht darin, daß der in den Dampfstrahlagglomerator integrierte Wirbelbetttrockner einen ringförmigen, konisch ansteigenden äußeren Fluidisierboden und einen zentralen inneren Fluidisierboden aufweist, wobei die Strömungsgeschwindigkeit der am inneren Boden austretenden Fluidisierluft größer ist als die Strömungsgeschwindigkeit der Fluidisierluft am äußeren Boden.

Vorteilhaft schließt sich an den konischen äußeren Fluidisierboden eine ebenfalls konische Erweiterung zum Agglomeratorgehäuse an. Diese Maßnahmen bewirken eine zirkulierende Bewegung des Bettes und verhindern eine unerwünschte örtliche Überfeuchtung. Die gewünschte Endfeuchte kann durch eine entsprechende Verweilzeit in der Trocknungszone und/oder entsprechende Wahl der Trocknungsluftmenge und -temperatur im Wirbelbett eingestellt werden. Die Temperatur im Wirbelbett liegt dabei zwischen 20° C und 70° C.

### Mit der Erfindung werden folgende Vorteile erzielt:

Mit dem Verfahren lassen sich aufgrund der geringen Agglomerierfeuchten, Temperaturbelastung und vor allem der sehr kurzen Belastungsdauer auch hydrolyseempfindliche Produkte erfolgreich durch Feuchtagglomeration verarbeiten. Es treten auch bei empfindlichen Produkten keine oder nur minimale Abbaureaktionen auf.
- Mit dem erfindungsgemäßen Verfahren können Agglomerate im Größenbereich zwischen 200 und 3000 µm, bevorzugt zwischen 200 und 2000 µm, hergestellt werden.
- Die mit dem Verfahren hergestellten Agglomerate sind extrem gut redispergierbar. Es werden auch für das Redispergieren in kaltem Wasser nur sehr kurze Zeiten benötigt (üblicherweise weniger als 1 Minute, bevorzugt < 30 Sekunden), die für Granulate, die mit üblicherweise angewendeten Agglomerationsverfahren (Mischeragglomeration, Wirbelschichtagglomeration) nur beim Redispergieren in heißem Wasser erreicht werden.
- Für schlecht schmeckende Produkte wird bei der Verarbeitung mit gut schmeckenden Produkten und / oder Aromen eine gute Geschmacksmaskierung erreicht.
- Bei der Wahl geeigneter Verfahrensbedingungen (insbesondere bei einem ausreichend hohen Anteil an Bindemittel) ist es möglich, unlösliche Produkte nicht nur zu agglomerieren, sondern auch teilweise zu umhüllen. Dabei wird die Oberfläche der unlöslichen Partikel mit dem gelösten Bindemittel überzogen, sofern die Bindemittellösungen auf dem unlöslichen Produkt spreitungsfähig sind. In diesem Fall kann eine Bedeckung der Oberfläche von 40 bis 80 %, bevorzugt von 50 bis 70 %, erreicht werden. Dies hat den Vorteil, daß die so umhüllten Produkte eine verminderte Reaktivität gegenüber anderen Mischungsbestandteilen aufweisen. So können auch Mischungen von Stoffen hergestellt werden, die normalerweise nur eine verminderte Lagerstabilität aufweisen.
- Die sehr gute Agglomeratlöslichkeit beruht darauf, daß die Brücken zwischen den zu agglomerierenden (unlöslichen) Partikeln aus einem sehr gut löslichen Material bestehen (Bindemittel löst sich in Sekundenbruchteilen im kondensierten Dampf!), das von Wasser auch sehr gut benetzt wird. Die Brücken zwischen den Partikeln sind darüber hinaus nur wenige µm dick (1 bis 30 µm, bevorzugt 5 bis 15 µm) und konzentrieren sich an den Kontaktstellen zwischen den Partikeln. So wird für einen Löseprozeß eine große Oberfläche angeboten.

Im folgenden wird die Erfindung an Hand von Ausführungsbeispielen und Zeichnungen näher erläutert. Es zeigen:
- Fig. 1: schematisch die Dampstrahl-Agglomerationsapparatur mit integriertem Wirbelbetttrockner
- Fig. 2: eine Draufsicht der Dampfstrahldüsen mit den Verteilerrohren
- Fig. 3: das Schutzrohr zur funktionellen Trennung von Befeuchtung und Trocknung des herabfallenden Produktschleiers
- Fig. 4: eine vergrößerte Darstellung des integrierten Wirbelbetttrockners.

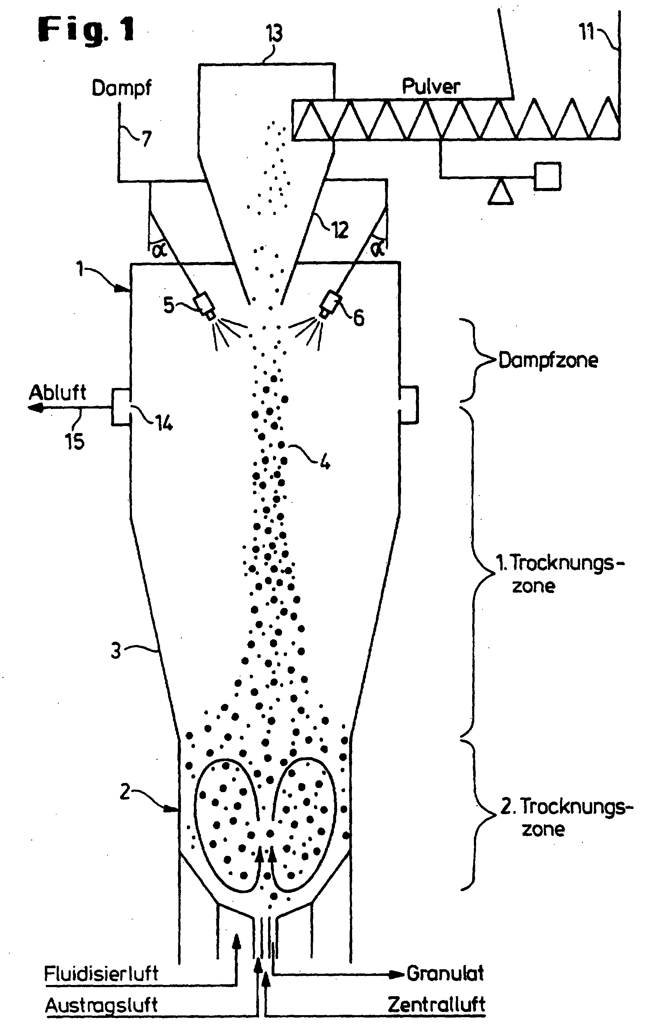

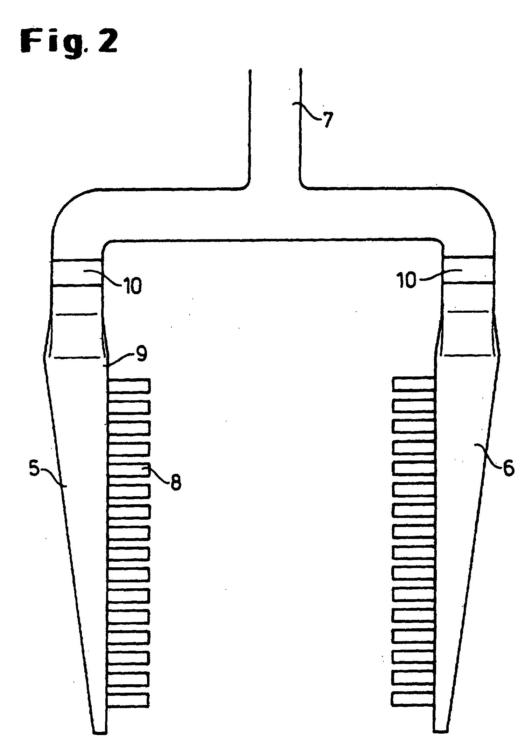

Gemäß Fig. 1 sind der Dampfstrahlagglomerator 1 und der Wirbelbettrockner 2 vertikal übereinander angeordnet und von demselben Gehäuse 3 eingeschlossen. Das den Dampfstrahlagglomerator 1 verlassende agglomerierte Produkt 4 fällt unmittelbar in den Wirbelbetttrockner 2. Die Dampfzufuhr erfolgt über zwei parallel geschaltete Dampfstrahldüsen 5 und 6, die zu beiden Seiten des herabfallenden Produktschleiers angeordnet sind. Diese werden über eine gemeinsame Dampfleitung 7 versorgt. Die Dampfstrahldüsen 5, 6 sind außerdem um den Winkel α schwenkbar. Wie in Fig. 2 gezeigt, bestehen die Dampfstrahldüsen 5, 6 jeweils aus einer Vielzahl von kurzen Rohren 8 oder Bohrungen, die mit einem sich in Längsrichtung des Agglomerators erstreckenden Verteilerrohr 9 verbunden sind. Der Querschnitt der Verteilerrohre 9 verringert sich in der Weise über ihre Länge, daß die Düsenrohre 8 mit gleichen Dampfmengenströmen beaufschlagt werden. Die Länge der Düsenrohre 8 beträgt mindestens das Dreifache ihres Durchmessers. Die Verteilerrohre 9 und damit auch die Düsenrohre 8 sind um den Drehpunkt 10 schwenkbar, so daß der Winkel, unter dem der Dampf auf den Produktschleier auftrifft, einstellbar ist. Der an den Dampfdüsen 5, 6 vorbei rieselnde Produktschleier wird innerhalb einer Dampfeinwirkungsstrecke d, deren Länge im wesentlichen durch die Austrittsgeschwindigkeit des Dampfes und den Schwenkungswinkel α bestimmt ist, mit dem Dampf beaufschlagt. Diese Dampfeinwirkungsstrecke wird im folgenden als 'Dampfzone' bezeichnet. Der Winkel α ist zwischen 0° und 60° einstellbar. Optimale Werte liegen zwischen 20° und 40°.

Die Feststoffzufuhr erfolgt über eine Dosierschnecke 11 in einen Längstrichter 12, an dessen unterem Ende ein gleichmäßiger pulverförmiger Produktschleier durch einen Schlitz austritt und in den Agglomerationsteil 1 gelangt. So werden die Partikel einander angenähert, aber es treten keine kompaktierenden Kräfte auf. Der Bereich über dem Feststoffeintrag ist durch einen separaten Deckel 13 ebenfalls abgeschlossen, so daß ein Eindringen von Falschluft in den Apparat vermieden wird bzw. nur eine kontrollierbare Luftmenge über den Produkteintrag in den Apparat gelangt. Zuviel Falschluft würde zu einer Störung der Dampfatmosphäre und zur Verschlechterung der Agglomerationswirkung führen.

Das zu agglomerierende Produkt und das Bindemittel werden der Dosierschnecke 11 als Mischung zugeführt. Das Produkt in der Vorlage kann mittels eines Heiz- oder Kühlmittels, z.B. durch konditionierte Luft, temperiert werden. Die Produkttemperatur, d.h. die Temperatur der Pulvermischung in der Dosierschnecke, ist ein einstellbarer Verfahrensparameter, der auf die kondensierende Menge Dampf und somit direkt auf das Agglomerierergebnis (Agglomeratgröße, Agglomeratfeuchte) Einfluß hat.

Eine wichtige Voraussetzung für die Erzielung reproduzierbarer Verfahrensbedingungen und damit gleichbleibendender Produktqualitäten ist die räumliche Abtrennung von Bedampfungs- und Trocknungszone, so daß beim Bedampfen eine nahezu reine Dampfatmosphäre gewährleistet ist. Zu diesem Zweck ist am Gehäuse 1 in Höhe der Stelle, an der die Dampfzone endet und die Trocknungszone beginnt, ein Ringspalt 14 angebracht, der mit einer Sammelleitung 15 verbunden ist. Durch diesen Ringspalt wird die gesamte, aus dem Wirbelbetttrockner 2 stammende Abluft abgezogen. In der Praxis befindet sich der Ringspalt 14 in einem Abstand von 50 mm bis 300 mm unterhalb der Unterkante der Verteilerrohre 9.

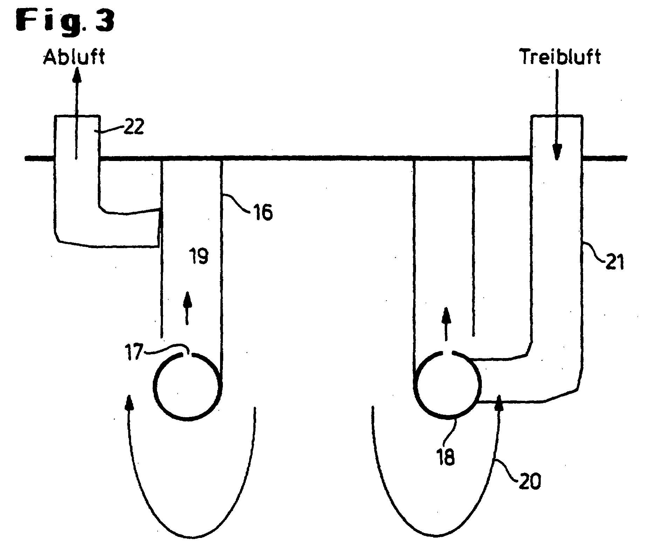

Eine alternative Möglichkeit zur räumlichen Abtrennung von Bedampfungs- und Trocknungszone beruht auf einem die Dampfzone umgebenden Schutzzylinder (s. Fig. 3). Der am Deckel 13 konzentrisch zum Gehäuse 1 angeordnete Schutzzylinder 16 ist doppelwandig ausgeführt. In diesen Doppelmantel wird von unten her durch einen kreisförmigen Schlitz 17 in einem Ringrohr 18 Heißluft eingeblasen, um den Schutzzylinder 16 zu beheizen und zu verhindern, daß an seinen Wänden Dampf kondensiert. Außerdem ist das Ringrohr 18 an dem Schubzylinder 16 so angebracht, daß zwischen dem Doppelmantel und dem Ringrohr 18 ein Ringspalt 19 verbleibt. Aufgrund der Injektorwirkung der eingeblasenen heißen Treibluft wird durch diese Öffnung am unteren Rand des Doppelmantels Luft aus dem Innenraum angesaugt (Pfeile 20), so daß ein gleichmäßiger Abzug der Abluft aus dem Trockner über den gesamten Umfang gewährleistet ist. Die heiße Treibluft wird dem Ringrohr 18 durch den Treibluftstutzen 21 zugeführt. Die Abluft wird aus dem Doppelmantel durch den Abluftstutzen 22 abgezogen.

In der Dampfzone wird der Produktschleier durch Kondensation befeuchtet und dadurch die Agglomeration eingeleitet. Nach dem Befeuchten fällt das agglomerierte feuchte Produkt zuerst durch einen entgegengesetzt strömenden Strom konditionierter, aus dem Wirbelbetttrockner stammender Trocknungsluft und wird dadurch vorgetrocknet. Die abschließende Trocknung auf die gewünschte Endfeuchte erfolgt in einem integrierten Fließbett oder Wirbelbett. Durch die Höhe des Fließbettes und durch die Wahl geeigneter Trocknungsbedingungen (Lufttemperatur, Luftmenge) wird die gewünschte Restfeuchte erzielt. Der Inhalt des Fließbettes wird mit Hilfe einer üblichen Füllstandsregelung auf einem gewünschten Wert konstant gehalten. Die Endfeuchte des Produktes kann somit unabhängig von der in der Dampfzone erreichten Agglomeratfeuchte genau eingestellt werden. Die Einstellung der gewünschten Restfeuchte erfolgt in jedem Fall erst dann, wenn die Bildung der Feststoffbrücken abgeschlossen ist. Die Granulatgröße und -struktur hängen von der Rezeptur und den gewählten Betriebsdaten (Verhältnis Feststoff-Dampfmenge, Feststofftemperatur, Trocknungsbedingungen) ab. Rückführende Feststoffkreisläufe, die zu Entmischungen führen können, sind nicht notwendig.

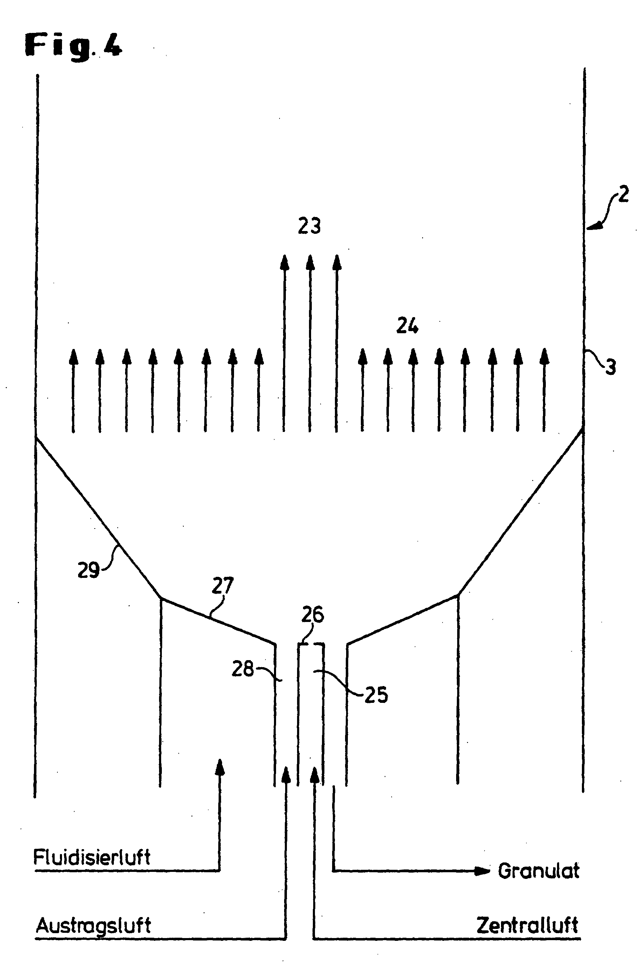

Gemäß Fig. 4 ist das Fließbett im Trockner 2 in verschiedene Bereiche unterschiedlich starker Fluidisierung aufgeteilt (Strömungspfeile 23 und 24). Im Zentrum wird das Bett durch ein separates Zentralrohr 25 mit einer Lochplatte 26 stärker fluidisiert als im peripheren Bereich, in dem die Fluidisierung in bekannter Weise durch einen konisch ausgeführten, nach außen hin ansteigenden Lochboden 27 erfolgt. Dadurch wird auftretendes noch feuchtes Produkt sofort unter bereits getrocknetes Produkt untergemischt und so an einer weiteren Granulierung bzw. am Zusammenbacken (Verkleben der bereits agglomerierten Teilchen untereinander) gehindert. So wird vermieden, daß die auf das Fließbett auftreffenden feuchten Agglomerate weiter agglomerieren, verkleben und zum Kollabieren des Fließbettes führen. Zwischen dem Lochboden 26 und dem Zentralrohr 25 ist ein ringförmiger Austragsspalt 28 angeordnet, durch den das getrocknete Endprodukt entsprechend dem Inhalt des Wirbelbettes, d.h. entsprechend seiner mittleren Verweilzeit im Apparat, ausgebracht wird. So ist auch gesichert, daß kein Produkt direkt, d.h. ohne vorherigen Umlauf durch das Fließbett, den Apparat verlassen kann. Direkt oberhalb des Lochbodens 27 erweitert sich der Apparat. Diese ebenfalls konische Erweiterung 29 (zum Gehäuse 1 hin) unterstützt die gleichmäßige Zirkulationsbewegung des Produktes und damit die Verteilung des auftreffenden, noch feuchten Agglomerates und fordert die Bewegung besonders grober Partikel hin zum Austrag 28. Durch die kombinierten Maßnahmen der gezielten unterschiedlichen Fluidisierung und der Erweiterung des Apparates wird ein besonders gleichmäßiger Produktumlauf gewährleistet.

Die erfindungsgemäße Dampftstrahl-Agglomerationsapparatur mit integriertem Wirbelbett wird mit folgenden Verfahrensparametern und Produktkenngrößen betrieben:

| **Mengenströme** | |
|---|---|
| Dampf | 5 bis 10 kg/h |
| Feststoff | 10 bis 100 kg/h |

| **Verweilzeiten** | |
|---|---|
| Dampfzone | 0,5 bis 3 s (bevorzugt 0,5 bis 1,5 s) |
| Fließbett | 10 bis 20 min |

| **Temperaturen** | |
|---|---|
| Dampf | 100 °C |
| Fluidisierluft | 20 bis 80 °C |
| Betttemperatur | 20 bis 50 °C |
| Produkt | 0 bis 60 °C |

| **Produktkenngrößen** | |
|---|---|
| Partikelgröße | < 300 µm (bevorzugt < 200 µm) |
| Bindemittelanteil | 5 bis 90 % |
| Agglomerat maximale Feuchte (nach der Dampfzone) | ca. 4 bis 5 % |
| Endfeuchte (nach der Fließbetttrocknung) | < 0,5 % |
| Agglomeratgröße | 200 bis 2000 µm |

### Ausführungsbeispiele

### Beispiel 1

100 g ASS-Pulver werden gemischt mit 50 g Xylitol als Bindemittel. Die Mischung, die Raumtemperatur hat, wird mittels der Schnecke 11 derart in den Agglomerationsapparat eingetragen, daß ein länglicher, gleichmäßiger Produktschleier entsteht. Der Feststoffmassestrom beträgt 20 kg/h. Er wird unter einem Winkel α = 30° mit 7 kg/h gesättigtem Wasserdampf bedüst. Die Verweilzeit in der Dampfzone und somit die Kontaktzeit mit dem Dampf beträgt 1 s. Dabei entstehen Granulate im Größenbereich bevorzugt zwischen 150 und 1000 µm. Die Granulate haben direkt nach Verlassen der Bedampfungszone Feuchten zwischen 1,5 % und 3 % (mit Hilfe der Karl-Fischer-Titrationsmethode bestimmt). Anschließend werden die Granulate im nachgeschalteten Fließbett bei Bettemperaturen von 40°C auf Restfeuchten unter 1 % getrocknet. Die Verweilzeit im Fließbett liegt zwischen 10 und 20 min. Das so hergestellte ASS-Xylitol-Granulat ist gut fließfähig, hat einen guten Geschmack und ist hervorragend redispergierbar. Der Gehalt an unerwünschten Abbauprodukten liegt unter 0,5 %.

### Beispiel 2

100 g ASS-Pulver werden gemischt mit 200 g Xylitol als Bindemittel. Die bei Raumtemperatur vorliegende Mischung wird mittels der Schnecke 11 derart in den Agglomerationsapparat eingetragen, daß ein länglicher, gleichmäßiger Produktschleier entsteht. Der Produktmassestrom beträgt 30 kg/h. Er wird unter einem Winkel α = 40° mit 10 kg/h Wasserdampf (gesättigt) bedüst. Es entstehen dabei Granulate im Größenbereich bevorzugt zwischen 150 und 1000 µm. Die Granulate haben direkt nach Verlassen der Bedampfungszone Feuchten zwischen 2,5 und 4 % (nach Karl-Fischer-Titration). Die Granulate werden im nachgeschalteten Fließbett auf Restfeuchten unter 1 % getrocknet. Das so hergestellte ASS-Xylitol-Granulat ist gut fließfähig, hat einen guten Geschmack und ist hervorragend redispergierbar. Der Gehalt an unerwünschten Abbauprodukten liegt unter 0,5 %. Die Oberfläche der ASS-Partikeln ist zu 50 bis 70 % mit Xylitol überzogen. So ist es möglich, die ASS gemeinsam mit anderen Komponenten zu mischen und zu lagern, mit denen eine gemeinsame Mischung bisher nicht möglich war, z.B. mit Brausebestandteilen oder anderen basischen Komponenten.

### Beispiel 3

100 g ASS-Pulver werden mit 30 g Saccharose und 5 g Aroma gemischt, auf 50°C vorgewärmt und mittels der Schnecke 11 derart in den Agglomerationsapparat eingetragen, daß ein länglicher, gleichmäßiger Produktschleier entsteht. Der Produktmassestrom beträgt 20 kg/h. Er wird unter einem Winkel α von 30° mit 6 kg/h Dampf bedüst. Es entstehen Granulate im Größenbereich bevorzugt zwischen 200 und 1400 µm. Die Granulate haben direkt nach Verlassen der Bedampfungszone Feuchten zwischen 1 und 2,5 % (nach Karl-Fischer-Titration). Die Granulate werden im nachgeschalteten Fließbett auf Restfeuchten unter 0,5 % getrocknet. Das so hergestellte Granulat ist gut fließfähig, hat einen guten Geschmack und ist hervorragend redispergierbar. Der Gehalt an unerwünschten Abbauprodukten der ASS liegt unter 0,5 %, der Verlust an üblicherweise flüchtigen Aromastoffen liegt unter 10 %. Auf diese Weise ist es möglich, ASS mit Aromen gemeinsam zu agglomerieren. Dies bietet den Vorteil, daß es gegenüber dem üblichen Vorgehen, Aromen nur zu diesem Zweck zu mischen, eine Entmischung im Verlaufe der weiteren Verarbeitung, des Transportes und der Lagerung bis hin zur Anwendung verhindert wird.

### Beispiel 4

100 g ASS werden mit 50 g Natriumsulfat gemischt und dampfstrahlagglomeriert, wie in Beispiel 1 beschrieben. Nach schonender Trocknung erhält man auch mit dieser Rezeptur ein gut fließfähiges, sehr schnell in Wasser dispergierbares Agglomerat, das für Sachet- oder Tablettenformulierungen angewendet werden kann.

### Beispiel 5

500 g Paracetamol werden gemeinsam mit 100 g Citronensäure, 400 g Orangenaroma, 1000 g Zitronenaroma sowie 2000 g Maltit als Bindemittel gemischt. Die Mischung wird bei Raumtemperatur mittels der Schnecke 11 derart in den Agglomerationsapparat eingetragen, daß ein länglicher, gleichmäßiger Produktschleier entsteht. Der Feststoffmassestrom beträgt 40 kg/h. Er wird unter einem Winkel α von 20° mit 10 kg/h Wasserdampf bedüst. Die Verweilzeit in der Dampfzone und somit die Kontaktzeit mit dem Dampf beträgt 1 s. Dabei entstehen Granulate im Größenbereich bevorzugt zwischen 200 und 2000 µm. Die Granulate haben direkt nach Verlassen der Bedampfungszone Feuchten zwischen 2 und 4 %. Anschließend werden die Granulate im nachgeschalteten Fließbett bei Bettemperaturen von 30°C auf Restfeuchten unter 0,5 % getrocknet. Die Verweilzeit im Fließbett liegt zwischen 15 und 20 min. Das so hergestellte Agglomerat ist gut fließfähig, hat einen guten Geschmack und ist hervorragend redispergierbar. Die Mengenverhältnisse zwischen den Komponenten nach der Agglomeration entsprechen dem bei der Ausgangsmischung eingestellten, d.h. es treten keine unerwünschten Entmischungserscheinungen auf. Das Agglomerat kann als Sachetformulierung oder auch als Tablettenformulierung dienen. Das Agglomerat löst sich in kaltem Wasser (5 g auf 100 ml) in einer Zeit von weniger als 15 s in ein anwendbares Getränk auf.

## Patentansprüche

1. Verfahren zur Agglomeration von schwer löslichen und hydrolyseempfindlichen Stoffen, **dadurch gekennzeichnet, daß** man Pulver des schwer löslichen Stoffes zusammen mit mindestens einem wasserlöslichen pulvrigen Bindemittel im freien Fall durch eine Wasserdampfatmossphäre bei Temperaturen zwischen 85°C und 105°C im wesentlichen ohne Einwirkung kompaktierender Kräfte führt mit einer Verweildauer in der Dampfzone von ca. 0,5 bis 10 Sekunden und anschließend im weiteren freien Fall antrocknet, so daß sich an den Kontaktstellen zwischen den Primärpartikeln aus den aufgrund der Kondensation entstandenen Flüssigkeitsbrücken, in denen Bindemittel gelöst ist, nur kleine Feststoffbrücken entstehen, die eine mittlere Querabmessung von 1 µm bis 30 µm, vorzugsweise 5 bis 15 µm, haben, und in einem nachfolgenden Trocknungsvorgang in einem integrierten Wirbelbett bis zu einem Wassergehalt von weniger als 5 Gew.-%, vorzugsweise weniger als 1 Gew.-%, trocknet.

2. Verfahren gemäß Anspruch 1, **dadurch gekennzeichnet, daß** als schwer löslicher hydrolyseempfindlicher Stoff ASS-Pulver eingesetzt wird.

3. Verfahren gemäß Anspruch 1, **dadurch gekennzeichnet, daß** die Verweildauer in der Wasserdampfzone ca. 1 bis 3 Sekunden beträgt.

4. Verfahren gemäß Anspruch 1, **dadurch gekennzeichnet, daß** man als gut wasserlösliche bindende Hilfsstoffe PVP, PVP-Derivate, Stärke, Stärke- und Zellulosederivate, Zucker, Zuckeralkohole, Zuckerderivate wie Maltodextrine, Isomaltose, Fruchtsäuren oder deren wasserlösliche Salze, Ascorbinsäure, Aminosäuren oder organische Salze oder Gemische dieser Stoffe verwendet.

5. Verfahren gemäß Anspruch 1, **dadurch gekennzeichnet, daß** das Mengenverhältnis der eingesetzten schwer löslichen Stoffe und der gut wasserlöslichen bindenden Hilfsstoffe 1 : 10 bis 10 : 1 beträgt.

6. Verfahren gemäß Anspruch 1, **dadurch gekennzeichnet, daß** man gut wasserlösliche anorganische Salze der Alkali- und Erdalkalireihe, wie z.B. Natriumchlorid, Natriumsulfat, Natriumcarbonat, -hydrogencarbonat, Magnesiumchlorid etc. verwendet.

7. Vorrichtung zur Durchführung des Verfahrens nach Ansprüchen 1 bis 6, ausgehend von einem Agglomerator (1) mit einem geschlossenen Gehäuse (3), an dessen oberen Ende eine Dosiervorrichtung (11) für ein pulverförmiges Gut in Verbindung mit einem Aufgabetrichter (12) angebracht ist, durch den ein im Agglomerator frei fallender Produktschleier von dem pulverförmigen Gut erzeugt wird, und mit Dampfstrahldüsen (5, 6) im oberen Teil des Agglomerators zur Erzeugung von Dampfstrahlen, die den frei fallenden Produktschleier innerhalb einer Dampfzone zumindest teilweise einhüllen, **dadurch gekennzeichnet, daß** sich an den unteren Teil des Agglomerators (1) ein Wirbelbetttrockner (2) derart anschließt, **daß** die agglomerierten Teilchen (4) direkt in das Wirbelbett fallen.

8. Vorrichtung nach Anspruch 7, dadurch gekenzeichnet, daß die Dampfstrahldüsen (5, 6) aus Rohren (8) oder Bohrungen bestehen, die mit einem sich in Längsrichtung des Agglomerators (1) erstreckenden Verteilerrohr (9) verbunden sind und daß zu beiden Seiten des Produktschleiers je ein Verteilerrohr (9) angeordnet ist.

9. Vorrichtung nach Ansprüchen 7 und 8, **dadurch gekennzeichnet, daß** im oberen Teil des Agglomerators (1) ein die Dampfstrahldüsen (5,6) einschließendes, beheiztes, doppelwandiges Schutzrohr (16) angeordnet ist, durch dessen Mantel Abluft aus dem Apparat abgezogen wird.

10. Vorrichtung nach Ansprüchen 7 und 8, **dadurch gekennzeichnet, daß** in einem Abstand von 50 mm bis 300 mm vom unteren Ende der Verteilerrohre (9) am Agglomeratorgehäuse (3) ein Ringspalt (14) mit einer Sammelleitung (15) zur Entnahme der Abluft vorgesehen ist.

11. Vorrichtung nach Ansprüchen 7 bis 10, **dadurch gekennzeichnet, daß** der Wirbelbetttrockner (2) einen ringförmigen, konisch ansteigenden äußeren Fluidisierboden (27) und einen zentralen inneren Fluidisierboden (26) aufweist und **daß** die Strömungsgeschwindigkeit der am inneren Boden (26) austretenden Fluidisierluft größer ist als die Strömungsgeschwindigkeit der Fluidisierluft am äußeren Boden (27).

12. Vorrichtung nach Anspruch 11, **dadurch gekennzeichnet, daß** sich an den konischen äußeren Fluidisierboden (27) eine ebenfalls konische Erweiterung (29) zum Agglomeratorgehäuse (3) hin anschließt.

## Claims

1. Process for the agglomeration of slightly soluble and hydrolytically sensitive substances, **characterized in that** powder of the slightly soluble substance is conducted, together with at least one water-soluble pulverulent binder, in free fall through a steam atmosphere at temperatures between 85°C and 105°C essentially without the action of compacting forces with a residence time in the steam zone of approximately 0.5 to 10 seconds and is then initially dried in further free fall so that, at the points of contact between the primary particles, only small solid bridges, which have a mean transverse dimension of 1 *µ*m to 30 *µ*m, preferably 5 to 15 *µ* m, are formed from the liquid bridges which are formed owing to the condensation and in which binder is dissolved, and is dried in a subsequent drying operation to a water content of less than 5 % by weight, preferably less than 1 % by weight, in an integrated fluidized bed.

2. Process according to Claim 1, **characterized in that** the slightly soluble hydrolytically sensitive substance used is ASA powder.

3. Process according to Claim 1, **characterized in that** the residence period in the steam zone is approximately 1 to 3 seconds.

4. Process according to Claim 1, **characterized in that**, as freely water-soluble binding auxiliaries, use is made of PVP, PVP derivatives, starch, starch and cellulose derivatives, sugar, sugar alcohols, sugar derivatives, such as maltodextrins, isomaltose, fruit acids or their water-soluble salts, ascorbic acid, amino acids or organic salts or mixtures of these substances.

5. Process according to Claim 1, **characterized in that** the ratio of the slightly soluble substances used and the freely water-soluble binding auxiliaries is 1:10 to 10:1.

6. Process according to Claim 1, **characterized in that** freely water-soluble inorganic salts of the alkali metal and alkaline earth metal series, such as sodium chloride, sodium sulphate, sodium carbonate, sodium hydrogen carbonate, magnesium chloride etc., are used.

7. Apparatus for carrying out the process according to Claims 1 to 6, starting from an agglomerator (1) having a closed housing (3), at the upper end of which a metering apparatus (11) for a pulverulent material connected to a feed hopper (12) is mounted, by which a freely falling product curtain of the pulverulent material in the agglomerator is produced, and having steam jet nozzles (5, 6) in the upper part of the agglomerator for producing steam jets which at least partially surround the freely falling product curtain within a steam zone, **characterized in that**, at the lower part of the agglomerator (1) a fluidized-bed dryer (2) is connected in such a manner that the agglomerated particles (4) fall directly into the fluidized bed.

8. Apparatus according to Claim 7, **characterized in that** the steam jet nozzles (5, 6) consist of tubes (8) or bore holes which are connected to a distributor tube (9) extending in the longitudinal direction of the agglomerator (1) and **in that** one distributor tube (9) is arranged on each of the two sides of the product curtain.

9. Apparatus according to Claims 7 and 8, **characterized in that** a heated double-walled protective tube (16) which encloses the steam jet nozzles (5, 6) is arranged in the upper part of the agglomerator (1), through the jacket of which protective tube (16) exhaust air is taken off from the apparatus.

10. Apparatus according to Claims 7 and 8, **characterized in that** a ring gap (14) having a collection line (15) for taking off the exhaust air is provided on the agglomerator housing (3) at a distance of 50 mm to 300 mm from the lower end of the distributor tubes (9).

11. Apparatus according to Claims 7 to 10, **characterized in that** the fluidized-bed dryer (2) has an annular, conically ascending outer fluidizing plate (27) and a central inner fluidizing plate (26) and **in that** the flow velocity of the fluidizing air exiting at the inner plate (26) is greater than the flow velocity of the fluidizing air at the outer plate (27).

12. Apparatus according to Claim 11, **characterized in that** a likewise conical widening (29) towards the agglomerator housing (3) adjoins the conical outer fluidizing plate (27).

## Revendications

1. Procédé d'agglomération de substances difficilement solubles et sensibles à l'hydrolyse, **caractérisé en ce qu'**on fait passer de la poudre de la substance difficilement soluble conjointement avec au moins un liant pulvérulent hydrosoluble en chute libre à travers une atmosphère de vapeur d'eau à des températures comprises entre 85°C et 105°C principalement sans l'action de forces de compactage, avec une durée de séjour dans la zone de vapeur d'environ 0,5 à 10 secondes, puis on la sèche, toujours en chute libre, de manière qu'il se formé aux endroits de contact entre les particules primaires à partir des ponts de liquide formés par condensation, dans lesquels le liant est dissous, uniquement de petits ponts de substance solide qui ont une dimension transversale moyenne de 1 µm à 30 µm, de préférence de 5 à 15 µm, et dans une opération de séchage subséquente, on effectue un séchage dans un lit tourbillonnaire intégré jusqu'à une teneur en eau de moins de 5 % en poids, de préférence de moins de 1 % en poids.

2. Procédé suivant la revendication 1, **caractérisé en ce qu'**on utilise, comme substance difficilement soluble, sensible à l'hydrolyse, de l'acide acétylsalicylique (ASS) en poudre.

3. Procédé suivant la revendication 1, **caractérisé en ce que** la durée de séjour dans la zone de vapeur d'eau est d'environ 1 à 3 secondes.

4. Procédé suivant la revendication 1, **caractérisé en ce qu'**on utilise, comme substances auxiliaires liantes se dissolvant bien dans l'eau, de la PVP (polyvinylpyrrolidone), des dérivés de PVP, de l'amidon, des dérivés d'amidon et de cellulose, des sucres, des alcools-sucres, des dérivés de sucre tels que maltodextrine, isomaltose, des acides de fruits ou leurs sels hydrosolubles, l'acide ascorbique, des amino-acides ou des sels organiques ou des mélanges de ces substances.

5. Procédé suivant la revendication 1, **caractérisé en ce que** le rapport quantitatif des substances difficilement solubles utilisées et des substances auxiliaires liantes se dissolvant bien dans l'eau va de 1:10 à 10:1.

6. Procédé suivant la revendication 1, **caractérisé en ce qu'**on utilise des sels inorganiques, se dissolvant bien dans l'eau, de la série des métaux alcalins et alcalinoterreux, par exemple chlorure de sodium, sulfate de sodium, carbonate de sodium, bicarbonate de sodium, chlorure de magnésium, etc.

7. Dispositif pour la mise en oeuvre du procédé suivant les revendications 1 à 6, constitué d'un agglomérateur (1) équipé d'un boîtier fermé (3) à l'extrémité supérieure duquel est adapté un dispositif doseur (11) pour une matière pulvérulente en liaison avec une trémie de chargement (12) par laquelle est formé un voile de la matière pulvérulente en chute libre dans l'agglomérateur, et avec des buses (5, 6) à jet de vapeur dans la partie supérieure de l'agglomérateur pour la production de jets de vapeur, qui enveloppent au moins en partie le voile de produit en chute libre à l'intérieur de la zone de vapeur, **caractérisé en ce qu'**un sécheur à lit tourbillonnaire (2) se raccorde à la partie inférieure de l'agglomérateur (1) de manière que les particules agglomérées (4) tombent directement dans le lit tourbillonnaire.

8. Dispositif suivant la revendication 7, **caractérisé en ce que** les buses (5, 6) à jets de vapeur sont constituées de tubes (8) ou d'orifices qui sont en liaison avec un tube répartiteur (9) s'étendant dans la direction longitudinale de l'agglomérateur (1) et **en ce qu'**un tube répartiteur (9) est disposé sur chacun des deux côtés du voile de produit.

9. Dispositif suivant les revendications 7 et 8, **caractérisé en ce qu'**un tube protecteur chauffé à double paroi (16), incluant les buses à jets de vapeur (5, 6) et à travers l'enveloppe duquel l'air d'échappement est chassé de l'appareil, est disposé dans la partie supérieure de l'agglomérateur (1).

10. Dispositif suivant les revendications 7 et 8, **caractérisé en ce qu'**un espace annulaire (14), comprenant un conduit collecteur (15) pour le prélèvement de l'air d'échappement est prévu à une distance de 50 mm à 300 mm de l'extrémité inférieure des tubes répartiteurs (9) sur le boîtier (3) de l'agglomérateur.

11. Dispositif suivant les revendications 7 à 10, **caractérisé en ce que** le sécheur (2) à lit tourbillonnaire présente un fond externe (27) de fluidisation de forme annulaire, s'élevant en cône, et un fond interne (26) de fluidisation à disposition centrale et **en ce que** la vitesse d'écoulement de l'air fluidisé sortant par le fond interne (26) est supérieure à la vitesse d'écoulement de l'air de fluidisation au niveau du fond externe (27).

12. Dispositif suivant la revendication 11, **caractérisé en ce qu'**un évasement (29) également en cône, dirigé vers le boîtier (3) de l'agglomérateur, se raccorde au fond externe conique (27) de fluidisation.
